(19) Europäisches Patentamt
European Patent Office
Office européen des brevets

(11) Veröffentlichungsnummer: **0 306 753 B1**

# (12) EUROPÄISCHE PATENTSCHRIFT

(45) Veröffentlichungstag der Patentschrift: **30.12.92**

(51) Int. Cl.$^5$: **A61K 7/13**

(21) Anmeldenummer: **88113538.8**

(22) Anmeldetag: **20.08.88**

## (54) Haarfärbemittel.

(30) Priorität: **28.08.87 DE 3728748**

(43) Veröffentlichungstag der Anmeldung:
**15.03.89 Patentblatt 89/11**

(45) Bekanntmachung des Hinweises auf die Patenterteilung:
**30.12.92 Patentblatt 92/53**

(84) Benannte Vertragsstaaten:
**AT BE CH DE ES FR GB GR IT LI LU NL**

(56) Entgegenhaltungen:
**EP-A- 0 176 798**
**EP-A- 0 195 361**
**DE-A- 294 184**
**DE-A- 2 659 056**
**US-A- 4 200 432**

**JOURNAL OF THE CHEMICAL SOCIETY, Teil 2, 1948, London (GB); ALBERT, Seiten 1228-1230**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Rose, David, Dr.**
**Holbeinweg 7**
**W-4010 Hilden(DE)**
Erfinder: **Lieske, Edgar**
**Hunsrückenstrasse 40**
**W-4000 Düsseldorf(DE)**
Erfinder: **Maak, Norbert, Dr.**
**Im Jagdfeld 41a**
**W-4040 Neuss(DE)**
Erfinder: **Höffkes, Horst, Dr.**
**Carlo-Schmid-Strasse 113**
**W-4000 Düsseldorf-Hellerhof(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Gegenstand der Erfindung sind Haarfärbemittel auf der Basis von Oxidationsfarbstoffen. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt, die unter dem Einfluß von Oxidationsmitteln oder von Luftsauerstoff Farbstoffe ausbilden. Als kosmetische Träger für die Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haaren spielen die sogenannten Oxidationsfarben, die durch oxidative Kupplung einer oder mehrerer Entwicklerkomponenten untereinander oder mit einer oder mehreren Kupplerkomponenten entstehen, wegen ihrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopryidinderivate, heterocyclische Hydrazonderivate, 4-Aminopyrazolonderivate und Tetraaminopyrimidine eingesetzt. Als sogenannte Kupplersubstanzen werden m-Phenylendiaminderivate, Naphthole, Resorcinderivate und Pyrazolone verwendet.

Gute Oxidationshaarfarbstoffvorprodukte müssen in erster Linie folgende Voraussetzungen erfüllen: Sie müssen bei der oxidativen Kupplung die gewünschten Farbnuancen in ausreichender Intensität ausbilden. Sie müssen ferner ein gutes Aufziehvermögen auf menschlichem Haar besitzen, ohne die Kopfhaut zu stark anzufärben. Auch soll der Farbaufzug gleichmäßig erfolgen, d.h. die stärker strapazierten Haarspitzen sollen nicht stärker gefärbt werden als der wenig geschädigte Haaransatz. Die damit erzeugten Färbungen müssen eine hohe Stabilität gegen Wärme, Licht und die bei der Dauerwellung des Haars verwendeten Chemikalien aufweisen. Schließlich sollen die Oxidationshaarfarbstoffvorprodukte in toxikologischer und dermatologischer Hinsicht unbedenklich sein.

Aminodiphenylamine anderer chemischer Struktur sind als Oxidationsfarbstoffvorprodukte z. B. aus DE-PS 294 184 und aus DE-OS 2 507 567 schon bekannt. Die mit diesen Produkten herstellbaren Haarfärbemittel befriedigen jedoch nicht in bezug auf die Echtheitseigenschaften der damit erzielbaren Haaranfärbungen. Insbesondere sind diese Aminodiphenylamine nicht als Rotkuppler für Entwicklerkomponenten vom Typ der 2,4,5,6-Tetraaminopyrimidine geeignet, da sie mit Entwicklern dieser Substanzklasse entweder keine roten Nuancen ausbilden oder weil die gebildeten Nuancen zu ungleichmäßig aufziehen und in der Regel die weniger geschädigten jüngeren Haaransatz-Bereiche weniger stark anfärben als die Haarspitzen. Diesen Nachteil weisen alle bekannten Rotkuppler für Entwickler des Tetraaminopyrimidin-Types auf.

Es wurde gefunden, daß Haarfärbemittel, enthaltend Oxidationsfarbstoffvorprodukte in einem Träger, die als Oxidationsfarbstoffvorprodukte Aminodiphenylamine der Formel (I)

(I)

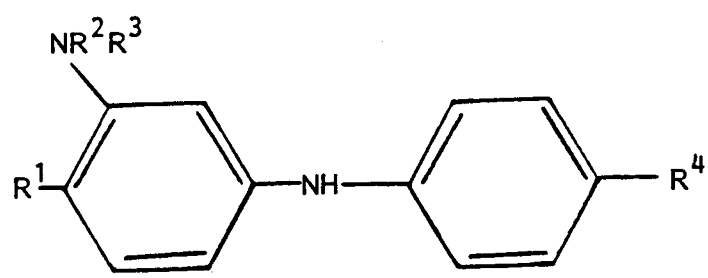

in der die Gruppe $R^1$ Wasserstoff, eine Alkylgruppe mit 1-4 C-Atomen oder ein Halogen, die Gruppen $R^2$ und $R^3$ unabhängig voneinander Wasserstoff, Alkylgruppen mit 1-4 C-Atomen oder Hydroxyalkylgruppen mit 2-4 C-Atomen oder gemeinsam mit dem Stickstoffatom einen Morpholin-, Piperidin-, Pyrrolidin- oder Piperazinring bilden und $R^4$ Wasserstoff oder eine Gruppe-$NR^2R^3$ ist, oder deren Salze als Kupplerkomponenten und die in Oxidationshaarfärbemittel üblichen Entwicklersubstanzen enthalten, die gestellten Anforderungen in hohem Maße erfüllen.

Die Aminodiphenylamine der Formel (I) sind als Kupplersubstanzen für eine Vielzahl bekannter Entwicklerverbindungen geeignet und erzeugen besonders brillante Färbungen mit hoher Licht- und Wärmestabilität. Als Entwicklersubstanzen können in den erfindungsgemäßen Haarfärbemitteln z.B. aromatische Amine mit einer oder mehreren weiteren $NH_2$-Gruppen, NHR-Gruppen, $NR_2$-Gruppen, wobei R eine Alkylgruppe mit 1-4 C-Atomen oder eine Hydroxyalkylgruppe oder eine Aminoalkylgruppe mit 2-4 C-Atomen darstellt, Aminophenole, Aminophenolether, Diaminopyridinderivate oder 2.4.5.6-Tetraaminopyrimidin und dessen Derivate eingesetzt werden.

Bevorzugt wegen ihrer guten Zugänglichkeit werden solche Aminodiphenylamine der Formel (I) eingesetzt, worin $R^1$ Wasserstoff, eine Methylgruppe oder Chlor, $R^2$ und $R^3$ unabhängig voneinander Wasserstoff oder eine Methylgruppe und $R^4$ Wasserstoff oder eine Aminogruppe ist.

Die Aminodiphenylamine der Formel (I) sind überwiegend literaturbekannt oder nach literaturbekannten Verfahren herstellbar. Die Herstellung des nicht literaturbekannten 3-Amino-4-chlordiphenylamin-hydrochlorid durch die sog. ULLMANN-Reaktion aus 4-Chlor-3-nitroacetanilid und Brombenzol in Gegenwart von Natriumcarbonat, Kaliumjodid und Kupferpulver in der Hitze und Reduktion der Nitrogruppe zur Aminogruppe wird in Beispiel 1 beschrieben. Nach dem gleichen Verfahren lassen sich auch andere Aminodiphenylamine der Formel (I) herstellen.

Die Aminodiphenylamine der Formel (I) stellen besonders wertvolle Kupperkomponenten für Entwickler vom Typ des 2,4,5,6-Tetraaminopyrimidins und dessen Derivate dar, da sie mit diesen Entwicklerkomponenten rote oder braunrote Nuancen ausbilden, die ein besonders gleichmäßiges Aufziehvermögen auf das Haar im Bereich des Haaransatzes und der Haarspitzen, d. h. ein besonders gutes Egalisiervermögen, aufweisen. Ein Vergleich des Egalisiervermögens eines erfindungsgemäßen Haarfärbemittels mit 5-Dimethylamino-4′-aminodiphenylamin als Kuppler und 2,4,5,6-Tetraaminopyrimidin als Entwickler mit dem eines sonst gleichen Haarfärbemittels mit dem als Rotkuppler für 2,4,5,6-Tetraaminopyrimidin bekannten 2-Methylresorcin zeigt, daß mit dem erfindungsgemäßen Haarfärbemittel die Farbdifferenz zwischen einer angefärbten, geschädigten Haarsträhne und einer angefärbten, ungeschädigten Haarsträhne auf weniger als die Hälfte des mit dem herkömmlichen Färbemittel mit 2-Methylresorcin als Kuppler erzielbaren Wertes verringert wird.

Ein besonders bevorzugter Erfindungsgegenstand sind daher erfindungsgemäße Haarfärbemittel, die als Entwicklersubstanz 2,4,5,6-Tetraaminopyrimidin oder dessen Derivate mit der Formel (II)

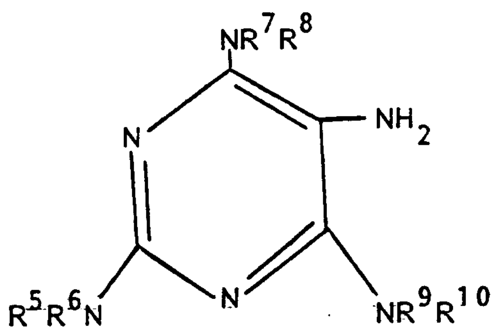

(II)

in der $R^5$-$R^{10}$ unabhängig voneinander Wasserstoff, Alkylgruppen mit 1-4 C-Atomen oder Hydroxyalkylgruppen mit 2-4 C-Atomen oder die Gruppen $R^5$ und $R^6$ bzw. $R^7$ und $R^8$ bzw. $R^9$ und $R^{10}$ jeweils gemeinsam mit dem Stickstoffatom einen Morpholin-, Piperidin-, Pyrrolidin- oder Piperazinring bilden oder deren Salze enthalten sind. Bevorzugt wird als Entwicklerkomponente der Formel (II) das 2,4,5,6-Tetraaminopyrimidin verwendet.

In die erfindungsgemäßen Haarfärbemittel können die Aminodiphenylamine der Formel I sowie die Tetraaminopyrimidine der Formel (II) entweder in freier Form oder auch in Form von Salzen der Aminogruppen mit anorganischen oder organischen Säuren, z.B. in Form der Hydrochloride, Sulfate, Phosphate, Acetate, Propionate, Lactate oder Citrate, eingesetzt werden.

Die erfindungsgemäßen Haarfärbemittel können neben den Aminodiphenylaminen der allgemeinen Formel (I) auch andere bekannte Kupplersubstanzen enthalten, die zur Modifizierung der Farbnuancen und zur Erzeugung natürlicher Farbtöne erforderlich sind. Solche üblichen Kupplerverbindungen sind z. B. andere m-Phenylendiamine, z. B. 2,4-Diaminophenyl-2-hydroxyethylether, 2,4-Diaminoanisol oder Phenole, Resorcine, m-Aminophenole, Naphthole oder Pyrazolone.

Zur Erzeugung natürlicher blonder, brauner oder schwarzer Haarfärbungen mit einem Oxidationshaarfärbemittel mit einem Entwickler vom Typ der 2,4,5,6-Tetraaminopyrimidine der Formel (II) ist es z. B. erforderlich, zusätzlich zu den Rotkupplern vom Typ der Aminodiphenylamine der Formel I noch Gelbkuppler und/oder Blaukuppler für diesen Entwickler einzusetzen, die ebenfalls mit Tetraaminopyrimidinen gut aufziehende Farbstoffe mit hohem Egalisierungsvermögen ausbilden. Besonders geeignete Gelbkuppler sind z. B. 2,7-Dihydroxynaphthalin und 2,6-Dihydroxy-3,4-dimethylpyridin. Ein besonders geeigneter Blaukuppler ist z. B. 2-Methyl-5-amino-6-chlorphenol.

Eine besonders bevorzugte Ausführungsform der Erfindung ist daher ein erfindungsgemäßes Haarfärbemittel mit einem 2,4,5,6-Tetraaminopyrimidin als Entwicklersubstanz, welches neben dem Aminodiphenylamin der Formel (I) als weitere Kuppler einen oder mehrere Kuppler aus der Gruppe 2,7-Dihydroxynaphtalin, 2,6-Dihydroxy-3,4-dimethylpyridin und/oder 2-Methyl-5-amino-6-chlorphenol enthält.

Gegebenenfalls können auch direktziehende Farbstoffe zusätzlich zur weiteren Modifizierung der Farbnuancen eingesetzt werden. Solche direktziehenden Farbstoffe sind z. B. Nitrophenylendiamine, Nitroaminophenole, Anthrachinonfarbstoffe oder Indophenole.

Zu den erfindungsgemäßen Haarfärbemitteln werden die Aminodiphenylamine der Formel (I) und die gegebenenfalls zusätzlich vorhandenen bekannten Kupplersubstanzen im allgemeinen in etwa molaren Mengen, bezogen auf die verwendeten Entwicklersubstanzen, eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklersubstanzen und Kupplersubstanzen in einem Molverhältnis von 1 : 0,5 bis 1 : 2 enthalten sein können.

Es ist nicht erforderlich, daß die Aminodiphenylamine der Formel (I) sowie die sonst in den Haarfärbemitteln vorhandenen Oxidationsfarbstoffvorprodukte oder direkt ziehenden Farbstoffe einheitliche chemische Verbindungen darstellen. Vielmehr können diese auch Gemische der erfindungsgemäß einzusetzenden Kuppler- oder Entwicklersubstanzen sein.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Zur Herstellung der erfindungsgemäßen Haarfärbemittel werden die Oxidationsfarbstoffvorprodukte in einen geeigneten kosmetischen Träger eingearbeitet. Solche Träger sind z. B. Cremes, Emulsionen, Gele oder auch tensidhaltige, schäumende Lösungen, z. B. Shampoos oder andere Zubereitungen, die für die Anwendung auf dem Haar geeignet sind. Übliche Bestandteile solcher kosmetischer Zubereitungen sind z. B. Netz- und Emulgiermittel wie anionische, nichtionische oder ampholytische Tenside, z. B. Seifen, Fettalkoholsulfate, Alkansulfonate, alpha-Olefinsulfonate, Fettalkoholpolyglykolethersulfate, Ethylenoxidanlagerungsprodukte an Fettalkohole, an Fettsäuren und an Alkylphenole, Sorbitanfettsäureester und Fettsäurepartialglyceride, Fettsäurealkanolamide sowie Verdickungsmittel wie z. B. Methyl- oder Hydroxyethylcellulose, Stärke, Fettkomponenten wie z. B. Fettalkohole, Paraffinöle oder Fettsäureester ferner Parfümöle und haarpflegende Zusätze, wie z. B. wasserlösliche kationische Polymere, Proteinderivate, Pantothensäure und Cholesterin.

Die Bestandteile der kosmetischen Träger werden zur Herstellung der erfindungsgemäßen Haarfärbemittel in für diese Zwecke üblichen Mengen eingesetzt, z. B. werden Emulgiermittel in Konzentrationen von 0,5 - 30 Gew.-% und Verdickungsmittel in Konzentrationen von 0,1 - 25 Gew.-% des gesamten Färbemittels eingesetzt.

Besonders geeignet als Träger ist eine Öl-in-Wasser-Emulsion mit einem Gehalt von 0,1 - 25 % Gew.-% einer Fettkomponente und 0,5 - 30 Gew.-% eines Emulgiermittels aus der Gruppe der anionischen, nichtionischen oder ampholytischen Tenside.

Die Oxidationsfarbstoffvorprodukte werden in Mengen von 0,2 - 5 Gew.-%, vorzugsweise 1 - 3 Gew.-% des gesamten Färbemittels in den Träger eingemischt. Der Gehalt an Aminodiphenylaminen Formel (I) kann in den erfindungsgemäßen Haarfärbemitteln etwa 0,05 - 10 Millimol pro 100 g des Haarfärbemittels betragen.

Die Anwendung der erfindungsgemäßen Haarfärbemittel kann, unabhängig von der Art der kosmetischen Zubereitung, z. B. als Creme, Gel oder Shampoo, bevorzugt im schwach alkalischen Milieu erfolgen. Bevorzugt ist die Anwendung der Haarfärbemittel in einem pH-Bereich von 8-10. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo verwendet wurde.

Die nachfolgenden Beispiele sollen den Erfindungsgegenstand näher erläutern.

## Beispiele

1. Herstellung von 3-Amino-4-chlor-diphenylamin-hydrochlorid (K 1)

Stufe 1: 3-Nitro-4-chlor-diphenylamin

Eine Mischung, bestehend aus 4-Chlor-3-nitroacetanilid (23 g) Brombenzol (34 g), Natriumcarbonat (8,1 g), Kaliumjodid (0,4 g) und Kupfer (Pulver, 0,24 g) in 125 ml Nitrobenzol wurde auf 180 °C-200 °C erwärmt und 18 Stunden bei dieser Temperatur gehalten. Nach Entfernung des Nitrobenzols durch Wasserdampfdestillation wurde der ölige Rückstand von der wäßrigen Phase abgetrennt und mit verdünnter Salzsäure zum Sieden erhitzt. Nach 3 Stunden wurde das Reaktionsgemisch auf kaltes Wasser gegossen und auf das ausfallende Produkt isoliert.

Stufe 2:

Das rote 3-Nitro-4-chlor-diphenylamin wurde in Ethanol in Gegenwart von katalytischen Mengen Ranay-Nickel mit Wasserstoffgas umgesetzt. Nach Beendigung der Wasserstoffaufnahme wurde die Lösung vom Katalysator abfiltriert und mit verdünnter Salzsäure angesäuert. Nach dem Einengen der Lösung zur Trockene wurden dunkelgraue Kristalle erhalten.

2. Anwendungsbeispiele

Es wurden erfindungsgemäße Haarfärbemittel in Form einer Haarfärbe-Cremeemulsion der folgenden Zusammensetzung hergestellt:

| | |
|---|---|
| Fettalkohol $C_{12}$-$C_{18}$ | 10 g |
| Fettalkohol $C_{12}$-$C_{14}$ + 2EO-sulfat, Na-Salz, 28%ig | 25 g |
| Wasser | 60 g |
| 1. Entwicklerkomponente (Komponente E) | 7,5 mMol |
| Aminodiphenylamin (Komponente K) | 7,5 mMol |
| $Na_2SO_3$ (Inhibitor) | 1,0 g |
| konzentrierte Amoniak-Lösung | bis pH = 9,5 |
| Wasser | ad 100 g |

Die Bestandteile wurden der Reihe nach miteinander vermischt. Nach Zugabe der Oxidationsfärbemittelvorprodukte und des Inhibitors wurde zunächst mit konzentrierter Amoniak-Lösung der pH-Wert der Emulsion auf 9,5 eingestellt, dann wurde mit Wasser auf 100 g aufgefüllt.

Die oxidative Entwicklung der Färbung wurde mit 3%iger Wasserstoffperoxidlösung als Oxidationsmittel durchgeführt. Hierzu wurden 100 g der Emulsion mit 50 g Wasserstoffperoxidlösung (3%ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Als Aminodiphenylamin (Komponente K) wurden die Produkte K1, K2, K3, K4 und K5 eingesetzt.

K1: 3-Amino-4-chlor-diphenylamin (Beispiel 1)
K2: 3-Amino-4-methyl-diphenylamin (bekannt aus DD-PS-80977)
K3: 3-Amino-diphenylamin (bekannt aus J.Chem.Soc.(1948),1228)
K4: 3-Dimethylamino-4′-aminodiphenylamin (bekannt aus J.Chem.Soc.(1948), 1229)
K5: 3,4′-Diamino diphenylamin (bekannt aus J.Chem.Soc. (1948), 1229

Als Entwicklerkomponente (Komponente E) wurden die folgenden Verbindungen eingesetzt:

E1: 2,4,5,6 - Tetraaminopyrimidin
E2: 2-Methylamino-4,5,6-triaminopyrimidin
E3: 2-Dimethylamino-4,5,6-triaminopyrimidin
E4: 2-Piperidyl-4,5,6-triaminopyrimidin
E5: 2-Morpholino-4,5,6-triaminopyrimidin
E6: 2.5.-Diaminotoluol

Die mit diesen Oxidationsfärbemittelvorprodukten in den Kombinationen gemäß Tabelle I enthaltenen Haaranfärbungen sind der Tabelle zu entnehmen.

Tabelle I

| Anwendungs-Beispiel | Entwickler-Komponente | Kuppler-Komponente | erhaltene Farbnuance |
|---|---|---|---|
| 2.1 | E1 | K1 | hellbraun |
| 2.2 | E4 | K1 | rotbraun |
| 2.3 | E5 | K1 | rotbraun |
| 2.4 | E1 | K2 | orangebraun |
| 2.5 | E2 | K2 | braunrot |
| 2.6 | E3 | K2 | braunrot |
| 2.7 | E4 | K2 | braunrot |
| 2.8 | E5 | K2 | braunrot |
| 2.9 | E1 | K3 | tizianrot |
| 2.10 | E2 | K3 | rotbraun |
| 2.11 | E1 | K4 | portweinrot |
| 2.12 | E1 | K5 | rotbraun |
| 2.13 | E6 | K1 | rubin |
| 2.14 | E6 | K2 | burgunderrot |
| 2.15 | E6 | K3 | schwarzviolett |
| 2.16 | E6 | K4 | blauviolett |
| 2.17 | E6 | K5 | schwarzblau |

## Patentansprüche

**1.** Haarfärbemittel, enthaltend Oxidationsfarbstoffvorprodukte in einem Träger, dadurch gekennzeichnet, daß als Oxidationsfarbstoffvorprodukte Aminodiphenylamine der Formel (I)

(I)

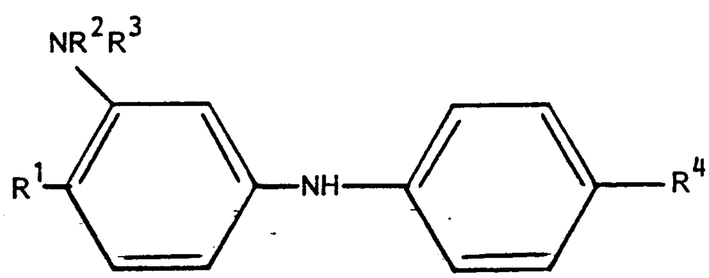

in der die Gruppe $R^1$ Wasserstoff, eine Alkylgruppe mit 1 bis 4 C-Atomen oder ein Halogen, die Gruppen $R^2$ und $R^3$ unabhängig voneinander Wasserstoff, Alkylgruppen mit 1 bis 4 C-Atomen oder Hydroxyalkylgruppen mit 2 bis 4 C-Atomen oder gemeinsam mit dem Stickstoffatom einen Morpholin-, Piperidin- oder Piperazinring bilden, und $R^4$ Wasserstoff oder eine Gruppe $-NR^2R^3$ ist, oder deren Salze als Kupplersubstanzen und die in Oxidationshaarfärbemitteln üblichen Entwicklersubstanzen enthalten sind.

**2.** Haarfärbemittel nach Anspruch 1, dadurch gekennzeichnet, daß als Kupplersubstanzen Aminodiphenylamine der Formel (I) nach Anspruch 1 enthalten sind, in der $R^1$ Wasserstoff, eine Methylgruppe oder Chlor, $R^2$ und $R^3$ unabhängig voneinander Wasserstoff oder eine Methylgruppe und $R^4$ Wasserstoff oder eine Aminogruppe ist.

**3.** Haarfärbemittel nach Anspruch 1, dadurch gekennzeichnet, daß als Entwicklersubstanz 2,4,5,6-Tetraaminopyrimidin oder deren Derivate mit der Formel (II)

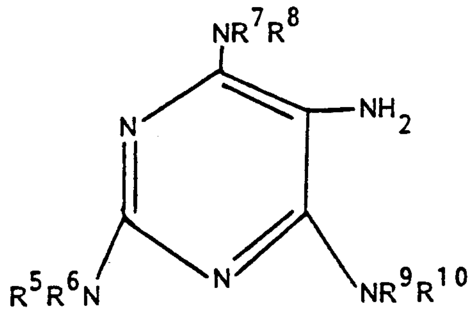

(II)

in der $R^5$ bis $R^{10}$ unabhängig voneinander Wasserstoff, Alkylgruppen mit 1-4 C-Atomen oder Hydroxyalkylgruppen mit 2-4 C-Atomen oder die Gruppen $R^5$ und $R^6$ bzw. $R^7$ und $R^8$ bzw. $R^9$ und $R^{10}$ jeweils gemeinsam mit dem Stickstoffatom einen Morpholin-, Piperidin-, Pyrrolidin- oder Piperazinring bilden oder deren Salze enthalten sind.

**4.** Haarfärbemittel nach Anspruch 3, dadurch gekennzeichnet, daß zusätzlich einer oder mehrere Kuppler aus der Gruppe 2,7-Dihydroxynaphthalin, 2,6-Dihydroxy-3,4-dimethylpyridin und/oder 2-Methyl-5-amino-6-chlorphenol enthalten sind.

**5.** Haarfärbemittel nach den Ansprüchen 1 bis 4, dadurch gekennzeichnet, daß als Träger eine Öl-in-Wasser-Emulsion mit einem Gehalt von 0,1 bis 25 Gew.-% einer Fettkomponente und 0,5 bis 30 Gew.-% eines Emulgiermittels aus der Gruppe der anionischen, nichtionischen oder ampholytischen Tenside und als Oxidationsfarbstoffvorprodukte Aminodiphenylamine der Formel (I) in einer Menge von 0,05 bis 10 Millimol pro 100 g des Haarfärbemittels enthalten sind.

**Claims**

**1.** Hair-dyeing preparations containing oxidation dye precursors in a carrier, characterized in that they contain as oxidation dye precursors aminodiphenylamines corresponding to formula (I)

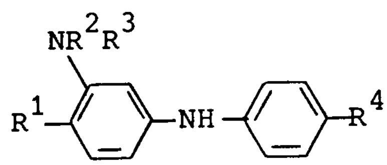

(I)

in which $R^1$ is hydrogen, a $C_{1-4}$ alkyl group or a halogen atom, $R^2$ and $R^3$ independently of one another represent hydrogen, $C_{1-4}$ alkyl groups or $C_{2-4}$ hydroxyalkyl groups or, together with the nitrogen atom, form a morpholine, piperidine or piperazine ring and $R^4$ is hydrogen or a group $-NR^2R^3$, or salts thereof as colour modifiers and the developer components normally used in oxidation hair dyes.

**2.** Hair-dyeing preparations as claimed in claim 1, characterized in that they contain as colour modifiers aminodiphenylamines corresponding to formula (I) in claim 1, in which $R^1$ is hydrogen, a methyl group or a chlorine atom, $R^2$ and $R^3$ independently of one another represent hydrogen or a methyl group and $R^4$ is hydrogen or an amino group.

**3.** Hair-dyeing preparations as claimed in claim 1, characterized in that they contain as developer component 2,4,5,6-tetraaminopyrimidine corresponding to the formula (II)

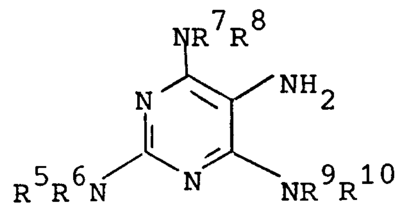

(II)

in which $R^5$ to $R^{10}$ independently of one another represent hydrogen, $C_{1-4}$ alkyl groups or $C_{2-4}$ hydroxyalkyl groups or the groups $R^5$ and $R^6$ or $R^7$ and $R^8$ or $R^9$ and $R^{10}$, together with the nitrogen atom, form a morpholine, piperidine, pyrrolidine or piperazine ring, or derivatives or salts thereof.

**4.** Hair-dyeing preparations as claimed in claim 3, characterized in that they additionally contain one or more colour modifiers from the group comprising 2,7-dihydroxynaphthalene, 2,6-dihydroxy-3,4-dimethyl pyridine and/or 2-methyl-5-amino-6-chlorophenol.

**5.** Hair-dyeing preparations as claimed in claims 1 to 4, characterized in that they contain an oil-in-water emulsion containing 0.1 to 25% by weight of a fatty component and 0.5 to 30% by weight of an emulsifier from the group of anionic, nonionic or ampholytic surfactants as carrier and aminodiphenylamines corresponding to formula (I) as oxidation dye precursors in a quantity of 0.05 to 10 millimol per 100 g of the hair-dyeing preparation.

**Revendications**

**1.** Teintures pour cheveux contenant des précurseurs de colorants d'oxydation dans un support, caractérisées en ce qu'elles renferment comme précurseurs de colorants d'oxydation, des aminodiphénylamines de formule (I)

( I )

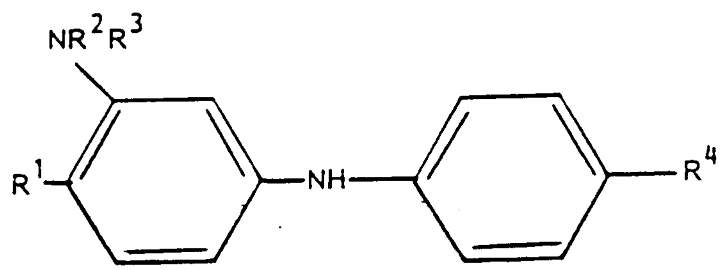

dans laquelle le groupe $R^1$ représente l'hydrogène, un groupe alkyle comportant 1 à 4 atomes de C ou un halogène, les groupes $R^2$ et $R^3$ représentent indépendamment l'un de l'autre l'hydrogène, des groupes alkyle possédant 1 à 4 atomes de C ou des groupes hydroxyalkyle comportant 2 à 4 atomes de C ou forment conjointement avec l'atome d'azote un cycle morpholine, pipéridine ou pipérazine, tandis que $R^4$ représente l'hydrogène ou un groupe $NR^2R^3$, ou leurs sels, à titre de copulants, ainsi que les substances révélatrices usuelles dans les teintures pour cheveux par oxydation.

**2.** Teintures pour cheveux selon la revendication 1, caractérisées en ce qu'elles renferment comme copulants, des aminodiphénylamines de la formule (I) selon la revendication 1, dans laquelle $R^1$ représente l'hydrogène, un groupe méthyle ou le chlore, $R^2$ et $R^3$ représentent indépendamment l'un de l'autre l'hydrogène ou un groupe méthyle, tandis que $R^4$ représente l'hydrogène ou un groupe amino.

**3.** Teintures pour cheveux selon la revendication 1, caractérisées en ce qu'elles renferment comme substance révélatrice de la 2,4,5,6-tétraaminopyrimidine ou ses dérivés de formule (II)

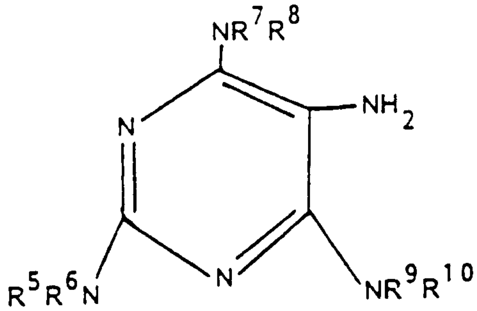

(II)

dans laquelle $R^5$ à $R^{10}$ représentent indépendamment l'un de l'autre l'hydrogène, des groupes alkyle comportant 1 à 4 atomes de C, des groupes hydroxyalkyle possédant 2 à 4 atomes de C ou les groupes $R^5$ et $R^6$ ou $R^7$ et $R^8$ ou $R^9$ et $R^{10}$ forment chaque fois conjointement avec l'atome d'azote un cycle morpholine, pipéridine, pyrrolidine ou pipérazine ou leurs sels.

**4.** Teintures pour cheveux selon la revendication 3, caractérisées en ce qu'elle renferment en plus un ou plusieurs copulants faisant partie du groupe du 2,7-dihydroxynaphtalène, de la 2,6-dihydroxy-3,4-diméthylpyridine et/ou du 2-méthyl-5-amino-6-chlorophénol.

**5.** Teintures pour cheveux selon les revendications 1 à 4, caractérisées en ce qu'elles renferment comme support, une émulsion huile dans l'eau contenant 0,1 à 25 % en poids d'un composant gras et 0,5 à 30 % en poids d'un émulsifiant appartenant au groupe des tensioactifs anioniques, non ioniques ou ampholytes et, comme précurseurs de colorants d'oxydation, des aminodiphénylamines de la formule (I) dans une proportion allant de 0,05 à 10 millimoles par 100 g de teinture.